# EUROPÄISCHE PATENTSCHRIFT

(11) **EP 1 888 004 B1**
(45) Veröffentlichungstag und Bekanntmachung des Hinweises auf die Patenterteilung: **29.04.2009**
(21) Anmeldenummer: 06742978.7
(22) Anmeldetag: 18.05.2006
(51) Int. Cl.: A61F 13/04

(54) **VERBANDMATERIAL SOWIE VERFAHREN ZU SEINER HERSTELLUNG**
DRESSING MATERIAL AND METHOD FOR ITS PRODUCTION
MATERIAU DE BANDAGE ET PROCEDE DE FABRICATION

(30) Priorität: 08.06.2005 DE 102005026298
(43) Veröffentlichungstag der Anmeldung: 20.02.2008
(73) Patentinhaber: Karl Otto Braun GmbH & Co. KG., 67752 Wolfstein (DE)
(72) Erfinder: LANGEN, Günter, 67752 Wolfstein (DE); MEISTER, Marita, 67657 Kaiserslautern (DE)
(74) Vertreter: Langöhrig, Angelika Beate
(86) Internationale Anmeldenummer: PCT/EP2006/004713
(87) Internationale Veröffentlichungsnummer: WO 2006/131196

(56) Entgegenhaltungen:
- EP-A- 1 029 521
- DE-A1- 3 544 151

## Beschreibung

Die Erfindung betrifft ein Verbandmaterial umfassend einen thermoplastischen Kunststoff, wobei der thermoplastische Kunststoff auf einer ersten Textilbahn aufgebracht ist.

Zur Herstellung von Verbänden zur Stützung und Fixierung von Gliedmaßen sind zum einen seit langem Gipsverbandmaterialien bekannt. Zum anderen wird seit einiger Zeit alternativ Verbandmaterial auf Kunststoffbasis verwendet. Dies besitzt gegenüber Gipsverbänden den Vorteil, dass es verbesserte mechanische Eigenschaften besitzt, insbesondere wasserunempfindlich und abwaschbar ist. Darüber hinaus lässt sich das Kunststoffmaterial schneller anlegen und aushärten und besitzt ein geringeres Gewicht, wodurch ein erhöhter Tragekomfort und dadurch eine verbesserte Mobilität gewährleistet ist. Schließlich besitzen Verbandmaterialien auf Kunststoffbasis den Vorteil gegenüber Gipsmaterialien für Röntgenstrahlen durchlässig zu sein und so Röntgennachkontrollen zuzulassen, ohne dass der Verband entfernt werden muss.

Sowohl Gipsverbandmaterialen als auch die auf Kunststoff basierenden Verbandmaterialen sind im Wesentlichen aus einem organischen oder anorganischen textilen Trägermaterial und dem darauf aufgebrachten Gips- beziehungsweise Kunststoffmaterial zusammengesetzt. Bei den Kunststoffmaterialien wird dabei zwischen irreversibel härtbaren Materialien und thermoplastischen, reversibel verformbaren Materialien unterschieden.

Als irreversible Kunststoffverbandmaterialien sind in erster Linie wasserhärtbare Systeme bekannt, die als härtbare Kunststoffkomponenten reaktive Polyurethanpräpolymere enthalten, die durch Kontakt mit Wasser aushärten. Solange der Kunststoff nicht ausgehärtet ist, sind die einzelnen Lagen des Verbandmaterials miteinander verklebbar, wodurch letztlich ein aus mehreren Schichten des Materials bestehender Verband erhalten wird.

Bei thermoplastischen reversibel verformbaren Verbandmaterialien wird die Selbstklebeeigenschaft durch Erwärmen des thermoplastischen Kunststoffs auf die jeweilige Schmelz- /Erweichungstemperatur oder darüber erzielt, so dass dann die Lagen beim Anwickeln eine permanente Verbindung miteinander eingehen. Beim Abkühlen erstarrt das Material wieder, wobei es auch bei Temperaturen unterhalb des Schmelzpunktes für einige Zeit plastisch modellierbar bleibt. Derartige Kunststoffe besitzen hinsichtlich ihres Schmelz- und Erstarrungsverhaltens einen Hystereseverlauf. Nach dem vollständigen Erstarren des thermoplastischen Kunststoffes wird ein stabiles, mehrlagig miteinander verbundenes Verbandsystem erhalten.

Ein entsprechendes Verbandmaterial ist beispielsweise aus der EP 1 029 521 A2 bekannt, die ein thermoplastisches, bei Temperaturen von 50 °C oder darunter starres oder semirigides und im plastischen Zustand selbstklebendes Verbandmaterial offenbart, das eine erste Textilbahn sowie einen auf die erste Textilbahn aufgebrachten thermoplastischen Kunststoff mit einem Schmelzpunkt von 55 °C bis 90 °C und mindestens eine auf diesen Materialverband aufgebrachte zweite Textilbahn umfasst. Hierdurch soll die Lagenhaftung am fertigen Verband nicht nachteilig beeinflusst werden, auf der anderen Seite soll die Binde jedoch leicht abrollbar sein, sogar nach Ausdrücken des Restwassers nach Erwärmen im Wasserbad. Ein Entfernen einer Schutzschicht ist dann nicht notwendig, so dass es zu einer deutlich leichteren Handhabbarkeit kommt. Schließlich wird auf diese Weise eine zweite nach außen gerichtete textile Oberfläche erzielt, die den Oberflächencharakter des fertigen Verbandes verbessern hilft. Um einen farbigen Verband zu schaffen, können Farbpigmente zugesetzt werden.

Nachteilig an der vorgestellten Gestaltung ist hierbei, dass die Farbpigmente unmittelbar mit dem Träger, aber auch dem Anwender beim Anlegen des Cast-Verbandes, in Kontakt kommen und durch toxische Bestandteile oder Abbauprodukte aus den Farbstoffen unerwünschte Hautreaktionen wie Hautreizung, Sensibilisierung, allergische Reaktion resultieren können. Dadurch besteht bei derartigen farbigen Castbinden das Problem, dass diese nicht als Dauerverband ohne zusätzliche Hautschutzmaterialien wie z.B. Polsterwatte, Schlauchverband, Krepppapier direkt auf der Haut getragen werden können.

Die Erfindung stellt sich daher die Aufgabe, ein Verbandmaterial bereitzustellen sowie ein Verfahren zur Herstellung eines entsprechenden Verbandmaterials, bei dem Farbvarianten eines entsprechenden Cast-Verbandes realisiert werden können, ohne dass bestimmte Farbpigmente die Haut von Anwender bzw. Patient unmittelbar berühren, so dass dadurch gleichzeitig vermieden wird, dass der Träger eines entsprechenden farbigen Cast-Verbandes mit möglicherweise toxischen Farbkomponenten in Kontakt kommt. Eine weitere Aufgabe der vorliegenden Erfindung besteht darin, farbige Castverbände zu realisieren, die auch ohne Verwendung von Hautschutzmaterialien wie z.B. Polsterwatte, Schlauchverband, Krepppapier über einige Wochen als Dauerverband direkt auf der Haut getragen werden können.

Die Erfindung löst diese Aufgabe durch ein Verbandmaterial nach Anspruch 1, ein Verfahren nach Anspruch 7, sowie eine Binde nach Anspruch 13 und eine Verwendung und einen orthopädischen Stützverband nach den Ansprüchen 12 und 14, bei dem der thermoplastische Kunststoff einen ersten mikroverkapselten Farbstoff enthält. Dadurch dass die Farbstoffe durch die Mikrokapselhülle eingeschlossen sind, wird erreicht, dass im eingefärbten Verbandstoff kein direkter Hautkontakt zu den Farbstoffen besteht und somit Hautreizungen durch toxische Farbkomponenten, wie insbesondere Azo-, Amin- oder Schwermetallkomponenten vermieden werden.

Entsprechende Farbstoff-Mikrokapseln, die insbesondere einen Partikeldurchmesser von 0,5 - 20 µm aufweisen können, bestehen aus einer dünnen Hülle, die zum Beispiel aus einem Aminoplast oder einem Harnstoff-Formaldehyd-Harz etc. bestehen kann. In die Hülle kann der Farbstoff direkt eingekapselt sein. Es kann auch vorgesehen sein, dass derartige Mikrokapseln neben dem Farbpigment und der Hülle auch noch eine Solvent- oder Wachskomponente aufweisen. Die Farbstoff-Mikrokapseln können dem thermoplastischen Kunststoff je nach gewünschter Farbtiefe in einem Gewichtsanteil zwischen 0,1 bis 8 Gew.-%, insbesondere 0,5 bis 4 Gew.-% zugesetzt werden.

Nach einem besonders bevorzugten Ausführungsbeispiel ist darüber hinaus vorgesehen, dass Mikrokapseln mit temperatursensiblen, sogenannten thermochromen Farbstoffen verwendet werden, die zum Beispiel bei Raumtemperatur einen intensiven Farbton, wie beispielsweise Rot, Blau, Grün etc. aufweisen und bei einer bestimmter Umschlagstemperatur Tu einen farblosen beziehungsweise transparenten Zustand einnehmen. Wird ein derartiger thermochromer Farbstoff für eine thermoplastische Binde eingesetzt, kann die Umschlagstemperatur des thermochromen Farbstoffs in gewissen Grenzen auf die Schmelzpunkt-/Erweichungstemperatur des thermoplastischen Kunststoffs eingestellt werden. Auf diese Weise wird das bisher bekannte Problem gelöst, dass thermoplastischen Verbandmaterialien der jeweilige Zustand, das heisst im wesentlichen der Applikationszustand, in dem der Verband thermisch aktiviert ist (Temperatur T größer als Tm = Schmelztemperatur des Kunststoffes) und somit plastisch verformbar ist, sowie der Funktionszustand, in dem der Verband im abgekühlten Zustand bei Raumtemperatur bzw. (Temperatur T kleiner als Tr = Erstarrungstemperatur des Kunststoffes) aufgrund der wieder gewonnenen Steifigkeit seine stützende Wirkung als Starrverband entfaltet, nicht direkt angesehen werden kann, was insbesondere bei der Nachbearbeitung von bereits angelegten Verbänden durch den Anwender wünschenswert wäre, aber auch über die gesamte Tragedauer des Verbands durch den Patienten. So kann es vorkommen, dass die Stützwirkung des Verbandes infolge von Sonneneinstrahlung oder anderweitiger Wärmeeinwirkung nachlässt. Eine Indikation des Zustandes wäre hier erstrebenswert.

Derartige thermochrome Farbstoffe, die mikroverkapselt sind, sind beispielsweise aus der DE 35 44 151 C2 vorbekannt.

Darüber hinaus offenbart auch die US-PS 5,412,035 bereits ein Verbandmaterial mit einem Klebstoff, der lediglich bei erhöhten Temperaturen als Haftschmelzklebstoff wirkt, bei Umgebungstemperatur jedoch nicht klebrig ist. Des Weiteren ist aus der Schrift bekannt, Zusatzstoffe vorzusehen, die entweder einen Farbumschlag signalisieren oder einen Wechsel von transparent nach opak, wenn der Klebstoff über eine gewisse vorgegebene Temperatur erhitzt oder darunter abgekühlt wird.

Erfindungsgemäß ist insbesondere der reversible Farbwechsel vorteilhaft. Weiterhin erfindungsgemäß ist, durch eine gezielte Auswahl der Wachs- beziehungsweise Solventkomponente innerhalb der mikroverkapselten Farbstoffe die Umschlagtemperatur Tu des thermochromen Farbstoffs auf eine beliebige Temperatur zwischen 20 °C und 80 °C einzustellen. Dies wird erreicht indem solche Solvent- beziehungsweise Wachskomponenten ausgewählt werden, die ihrerseits einen Schmelz- oder Erweichungspunkt aufweisen, der dem gewünschten Umschlagspunkt des thermochromen Farbstoffs entspricht. Insbesondere ist es möglich die Umschlagtemperatur Tu des thermochromen Farbstoffes auf den Schmelzpunkt Tm oder Erstarrungspunkt Tr des thermoplastischen Kunststoffes abzustimmen. Genauso ist es möglich den Farbumschlag auf den Hysteresebereich ΔH_{K}, den Funktionszustand oder den Applikationszustand des thermoplastischen Kunststoffes zu richten. Hierzu werden solche Wachs- beziehungsweise Solventkomponenten ausgewählt, die einen Schmelz- oder Erweichungspunkt aufweisen, die dem Schmelzpunkt Tm oder Erstarrungspunkt Tr des thermoplastischen Kunststoffes entsprechen. Es kann jedoch auch vorgesehen sein, solche Wachs- beziehungsweise Solventkomponenten auszuwählen, die einen Schmelz- oder Erweichungspunkt aufweisen, der innerhalb des Temperaturintervalls des Applikationszustandes oder innerhalb des Temperaturintervalls des Funktionszustandes liegt. Dabei werden vorteilhaft solche reversiblen Farbwechsel bevorzugt, die bei Erwärmung einen Umschlag von einem intensiven Farbton in einen insbesondere farblosen oder transparenten Farbton erzielen.

Dieser Farbumschlag signalisiert dem Anwender die richtige Aktivierung als Funktion von Temperatur und Zeitdauer der Lagerung des Verbandmaterials in einem Heizgerät, beispielsweise einem Wasserbad, Wärmeschrank oder dergleichen, zur Aufwärmung. Während der Applikation des Verbandmaterials kehrt infolge der Abkühlung des Thermoplasten der ursprüngliche Farbton unterhalb der Umschlagstemperatur Tu des thermochromen Farbstoffes wieder zurück und signalisiert dem Anwender beziehungsweise Patienten die Verfestigung und Erstarrung des Verbandmaterials und damit die gewünschte Stützwirkung. Der temperatursensible Farbwechseleffekt erfolgt reversibel - innerhalb eines Hysteresebereichs ΔH_{F} um die Umschlagstemperatur Tu - und begleitet jeden Aktivierungs- und Erstarrungszyklus, zum Beispiel auch bei partiellen Nachverformungen mittels Heißluft, Fön etc. Die Einstellung des Umschlagzeitpunktes erfolgt dabei über die Solvent- bzw. Wachskomponente der Farbstoffmikrokapseln und ist im weitesten Sinne zwischen 20° und 80° C in Abhängigkeit vom Schmelzpunkt der Solvent- bzw. Wachszubereitung in den Mikrokapseln beliebig einstellbar. Dabei kann vorgesehen sein, dass der Farbumschlag auf die Temperatur der Phasenumwandlung des thermoplastischen Kunststoffes beziehungsweise der Schmelz- bzw. Rekristallisationstemperatur abgestimmt ist und damit mit den thermischen sowie mechanischen, insbesondere viskoelastischen Materialparametern (Viskosität, Schermodul) des thermoplastischen Kunststoffes korreliert. Die Phasenumwandlung des thermoplastischen Kunststoffes ist durch einen mehr oder weniger ausgeprägten Hysteresebereich ΔH_{K}, d.h. Differenz zwischen Schmelz- und Erstarrungspunkt gekennzeichnet.

Generell wird in den Mikrokapseln durch die Aufwärmung des Wachsmaterials und dessen Überführung in den flüssigen Zustand eine Trennung zweier die Farbgebung gemeinsam erzeugender Komponenten erzielt, die erst wieder bei der Verfestigung des Wachsmaterials miteinander in Kontakt kommen und dann über Elektroneninteraktion eine sichtbare Farbe erzeugen.

Der Hysteresebereich ΔH_{F} der mikroverkapselten thermochromen Farbstoffe liegt dabei in einem Intervall von 1 bis ca. 10 K. Beim Aufwärmen ist der Farbtonwechsel am oberen Temperaturpunkt vollständig abgeschlossen, beim Abkühlen entsprechend erst am unteren Temperaturpunkt des Hysteresebereichs. Dagegen weisen die thermoplastischen Kunststoffe in aller Regel einen breiteren Hysteresebereich ΔH_{K} auf, der ca. 10 bis 40 K umfassen kann. Wegen dieses breiten Bereichs der Phasenumwandlung wird es erst möglich, dass sich innerhalb eines akzeptablen Zeitfensters die Verbandmaterialen verarbeiten lassen.

Erfindungsgemäß kann in einer weiteren Ausführung vorgesehen sein, dass ein weiterer Farbstoff beinhaltet sein kann. Bei diesem weiteren Farbstoff kann es sich um einen nicht mikroverkapselten Farbstoff oder insbesondere um einen weiteren mikroverkapselten und insbesondere einen weiteren thermochromen Farbstoff handeln. Insbesondere können Mischungen verschiedenfarbiger mikroverkapselter Farbstoffe mit oder ohne thermochrome Eigenschaften eingesetzt werden, um vielfältige Farbwechsel beziehungsweise Farbtöne einzustellen. So kann eine besondere Ausführungsform zum Beispiel vorsehen, dass das Verbandmaterial im kalten Zustand eine grüne Färbung aufweist und im aktivierten Zustand eine gelbe Färbung besitzt. Insbesondere können auch mehrere Arten von mikroverkapselten thermochromen Farbstoffen eingesetzt werden, die unterschiedliche Umschlagstemperaturen besitzen. Über den stufenweisen Farbwechsel je nach momentaner Temperatur kann das Aufwärmen beziehungsweise Abkühlen noch differenzierter beurteilt werden. Diese optische Signalwirkung ermöglicht es sowohl dem Anwender als auch dem Patienten, die Erstarrungsphase und somit die noch verbleibende Bearbeitungszeit beziehungsweise einem Patienten die momentane Stützwirkung und Belastbarkeit des Verbandmaterials genau zu beurteilen und gegebenenfalls Folgeverletzungen zu vermeiden. Insbesondere kann hierüber auch eine Signalwirkung dahingehend erzielt werden, dass zunächst ein erster Farbwechsel bei Erreichen eines Bearbeitungszustandes erfolgt und dann ein zweiter Temperaturwechsel signalisiert, dass nun das aufgeheizte Verbandmaterial einen Temperaturzustand erreicht hat, der für den Patienten als unangenehm zu bewerten ist.

Darüber hinaus können durch die Hinzufügung verschiedener thermochromer Farbstoffe interessante optische Effekte erzielt werden, indem die Farbwechsel so eingestellt werden, dass bei Veränderung der Umgebungstemperatur eine Farbänderung ähnlich einem Chamäleon-Effekt durch den Cast-Verband erfolgt.

Insbesondere kann vorgesehen sein, dass weitere Textilbahnen und/oder weitere thermoplastische Kunststoffschichten vorgesehen sind und die verschiedenen Schichten zu einem Verbund angeordnet sind. Als thermoplastische Kunststoffe kommen grundsätzlich solche in Frage, die bei Temperaturen von 40 °C, insbesondere von 50 °C oder darunter starr oder semirigide sind. Insbesondere können als thermoplastische Kunststoff auch Mischungen verschiedener Kunststoffe eingesetzt werden, soweit die Kunststoffe miteinander mischbar sind. Insbesondere kann als Kunststoff ein Hotmelt-Klebstoff, der bei Temperaturen von 55 °C - 90 °C, vorzugsweise von 60 °C - 80 °C und insbesondere bevorzugt von 60 °C - 70 °C schmilzt und auch nach Abkühlung unter den Schmelzpunkt für einige Zeit plastisch bleibt, eingesetzt werden. Damit der thermoplastische Kunststoff in einem thermoplastischen Verbandmaterial unter den normalen Anwendungsbedingungen einsetzbar ist, muss er eine Temperaturbeständigkeit bis 40 °C, vorzugsweise bis 50 °C und insbesondere vorzugsweise bis 55 °C aufweisen und es darf bei diesen Temperaturen nicht zu einer wesentlichen Erweichung oder zu einer Zersetzung des Kunststoffs kommen. Vorzugsweise hat der thermoplastische Kunststoff einen Schmelzindex bei 125 °C von 0,5 - 200 g/10 min, weiter bevorzugt 4 - 40 g/10 min, besonders bevorzugt von 12 - 25 g/10 min, wobei die Bestimmung des Schmelzindexes gemäß DIN ISO 1133 bei einer Prüftemperatur von 125 °C und einer Nominallast von 325 g erfolgt. Die Aushärtzeit nach dem Erwärmen auf oder über den Schmelzpunkt hängt von der erreichten Temperatur und der Abkühlgeschwindigkeit ab und beträgt im Allgemeinen 1 - 15 min, vorzugsweise 2 - 10 min und bevorzugt 3 - 8 min. Geeignete thermoplastische Kunststoffe mit den beschriebenen Eigenschaften sind beispielsweise Polyester, Polyurethan, Polyvinylacetat oder auch lineare gesättigte Polyesterverbindungen. Ein solcher Polyester ist kommerziell unter dem Namen "Polycaprolacton Capa" über die Fa. Solvay Interox, Warrington, UK erhältlich. Darüber hinaus können die thermoplastischen Polymere auch weitere Hilfs- oder Zusatzstoffe wie Stabilisatoren, Weichmacher, Harze, Tackifyer, UV-Filter, Füllstoffe oder Antioxidationsmittel enthalten. Weitere thermoplastische Kunststoffe, die eine gewisse Restflexibilität aufweisen können, sind zum Beispiel Polyethylenacrylsäureester-Copolymere, Ethyl-VinylacetatCopolymere und Polyurethane. In diesem Fall wird keine vollständige Fixierung eines mit einem Verband zu versehenen Körperteils bewirkt, sondern es verbleibt eine gewisse Restbeweglichkeit, die eine funktionelle Belastung der betroffenen Körperteile ermöglicht.

Durch die Vorsehung einer zweiten Textillage auf der Kunststoffschicht kann insbesondere erreicht werden, dass eine entsprechende Cast-Binde leicht abrollbar ist, auch nach Erwärmen und gegebenenfalls Ausdrücken in einem Wasserbad. Darüber hinaus kann auf die Vorsehung einer Verklebungsschutzschicht verzichtet werden und die Binde kann unmittelbar aufeinander aufgerollt werden. Darüber hinaus wird durch die Entstehung einer textilen Oberflächencharakteristik der Tragekomfort erhöht. Als textile Materialien für die erste oder auch weitere Textilbahnen kommen grundsätzlich elastische, aber auch nicht elastische Fasern und Fasermaterialien in Betracht. Dabei können sowohl synthetische als auch natürliche Fasern zum Einsatz kommen. Als Textilbahnen können textile Vliesbahnen, aber auch Gelege oder Gewirke sowie Gewebe oder gestrickte Textilien eingesetzt werden. Als thermochrome Farbstoffe können zum Beispiel die mikroverkapselten Chromazone-Pulver der Firma TMC - Thermographic Measurements Ltd., Flintshire (UK) eingesetzt werden.

Weiterhin umfasst die Erfindung eine Binde, die ein vorstehend beschriebenes Verbandmaterial umfasst. Die Binde kann dabei insbesondere aus dem Verbandmaterial durch Aufwickeln hergestellt sein. Auch umfasst die Erfindung die Verwendung eines vorstehenden Verbandmaterials für die Herstellung eines orthopädischen Stützverbandes sowie einen Stützverband.

Schließlich umfasst die Erfindung ein Verfahren zum Herstellen eines Verbandmaterials mit den Schritten: Bereitstellen einer Textilbahn, Mischen eines thermoplastischen Kunststoff mit einem mindestens ersten mikroverkapselten Farbstoff und/oder einer Farbzubereitung umfassend einen ersten mikroverkapselten Farbstoff zur Herstellung einer Beschichtungsmasse, Beschichten der Textilbahn mit der Beschichtungsmasse, vorzugsweise Auflegen einer weiteren Textilbahn auf die Beschichtungsmasse und Konfektionieren des Verbandmaterials, insbesondere durch Schneiden in bahnförmiges Material vorgegebener Länge und Breite bzw. Wickeln von Rollen vorgegebener Breite und Durchmesser. Hierdurch kann eine Binde erhalten werden. Insbesondere kann vorgesehen sein, dass vor dem Konfektionieren ein Abkühlschritt erfolgt. Zum Beschichten der Textilbahn kann darüber hinaus ein Erhitzen erforderlich sein und insbesondere wird ein Extrudierverfahren eingesetzt.

Weiterhin umfasst das Verfahren, dass die Farbzubereitung durch Zugabe eines mikroverkapselten Farbstoffs zu einem Trägerkunststoff hergestellt wird, wobei der Trägerkunststoff einen Schmelzpunkt T_{B} im Bereich von 10 K unterhalb bis 60 K oberhalb des Schmelzpunkts Tm des zur Herstellung des thermoplastischen Verbandmaterials verwendeten Kunststoffes aufweist.

Insbesondere kann als Trägerkunststoff der identische thermoplastischen Kunststoff eingesetzt werden. Die Mischung aus Trägerkunststoff und mikroverkapseltem Farbstoff wird geschmolzen, homogenisiert und nach dem Abkühlen granuliert. Eine derartige Farbzubereitung wird allgemein als Master-Batch bezeichnet. Der Master-Batch kann dabei weitere Zuschlagstoffe wie Füllstoffe, Gleitmittel, Stabilisatoren und Verarbeitungshilfen enthalten.

Alternativ ist es möglich, eine sogenannte Flüssigfarbe herzustellen, bei der die mikroverkapselten Farbstoffe zu einer inerten Flüssigkeit wie insbesondere einem Mineralöl, einem Esteröl, einem Fettsäureester, Fettalkohol oder Polyethylenglykolen zugemischt wird. Insbesondere können Monocarbonsäureester als flüssige gesättigte Fettsäure-Fettalkoholester, zum Beispiel n-Hexyllaurat oder n-Octylcaprylat, sowie wachsartige gesättigte Fettsäure-Fettalkoholester, zum Beispiel Talgalkoholpalmitatstearat oder Pentaerythritester, verwendet werden. Hierbei können ebenfalls Hilfsstoffe wie Tenside oder Stabilisatoren sowie Thixotropiermittel vorgesehen sein, wobei aus der inerten Flüssigkeit und dem mikroverkapselten Farbstoff, der in Pulverform vorliegt, eine homogene Dispersion hergestellt wird. Dabei soll der mikroverkapselte Farbstoff in möglichst fein verteilter Form frei von Pigmentaggregation in der Flüssigkeit vorliegen. Dabei ist insbesondere darauf zu achten, dass die Mikrokapseln nicht beschädigt und damit in ihrer Farbwechselfunktion beeinträchtigt werden. Der Gewichtsanteil der Farbstoffmikrokapseln kann zwischen 20 und 70 % eingestellt werden.

Die Erfindung soll im Folgenden anhand von Beispielen näher erläutert werden. Erfindungsgemäß wird dabei bei der Herstellung des thermoplastischen Verbandmaterials einem thermoplastischen Kunststoff mit geeignetem Schmelz- und Erstarrungspunkt ein wärmesensitiver mikroverkapselter Farbstoff zugesetzt, so dass der Farbstoff möglichst homogen und feindispers in der Kunststoffmatrix verteilt ist. Die Herstellung des thermoplastischen Verbandmaterials lässt sich dabei der DE 199 07 043 B4 entnehmen, und es wird hierauf explizit Bezug genommen.

Im Nachfolgenden soll daher die Herstellung des thermoplastischen Kunststoffmaterials, das dann zur Beschichtung oder Imprägnierung oder anders gearteter Verbindung mit dem Textilmaterial eingesetzt wird, erläutert werden. Um eine genügende Feinverteilung der Mikrokapseln in der Kunststoffmatrix und damit eine zufriedenstellende Farbhomogenität zu erreichen, werden die Mikrokapseln vorzugsweise nicht direkt in den schmelzflüssigen Kunststoff eingerührt beziehungsweise als Pulver im Extruder zugesetzt, sondern in einem vorgelagerten Schritt zunächst in einem sogenannten Master-Batch (konzentrierte Mikrokapseln/Kunststoffmischung mit 20 - 50 % Mikrokapselanteil) verarbeitet oder aber als Flüssigfarbzubereitung (Aufschlemmung der Mikrokapseln in geeigneten inerten Flüssigkeiten mit 30 - 70 % Mikrokapselanteil) bereitgestellt.

### Beispiel 1: Dosierung über Masterbatch

Ein Masterbatch besteht generell aus dem Trägerkunststoff, dem Farbpigment und (als Option) Zuschlagstoffen wie Füllstoffe, Gleitmittel, Stabilisatoren, Verarbeitungshilfen. Als Trägerkunststoff zur Herstellung des Mikrokapsel-Farbstoff-Masterbatches wird im Beispiel der gleiche thermoplastische Kunststoff verwendet, der auch zur Herstellung des thermoplastischen Verbandmaterials dient.

Dabei werden 50 Gewichtsteile Polycaprolacton der Firma Solvay Interox, Warrington, UK (CAPA 640, Schmelzpunkt 57 °C) als weisses Granulat (Pellets mit 4mm Korngröße) bei Raumtemperatur in einem Feststoffmischer vorgelegt und mit 50 Gewichtsteilen eines mikroverkapselten Blaufarbstoffs (CHROMAZONE - Pulver der Firma TMC - Thermographic Measurements Ltd., Flintshire (UK), Umschlagtemperatur 47 °C , Hysteresebereich 5,4 K ) homogen vermischt. Zur Verbesserung der Homogenisierung kann 1 bis 2 g Gleitmittel in Form von Wachsen, Ölen, Esterölen sowie Antistatikmittel zusätzlich zugegeben werden. Die homogenisierte Mischung wird über Trichter und Einzugswerk in einem Einschnecken- oder Doppelschneckenextruder bei 100 °C aufgeschmolzen und durch geeignete Rühr-, Misch- und Fördersegmente in der Schnecke im Extruder weiter homogenisiert. Da die Temperatur der Schmelze oberhalb der Umschlagtemperatur des mikroverkapselten thermochromen Farbstoffs liegt, tritt am Ende des Extruders eine farblose Kunststoff-Schmelze aus, die durch eine Runddüse (Durchmesser 3 mm) zu einem Endlosstrang geformt wird, der zur Abkühlung sofort in ein Wasserbad mit Kaltwasser geführt wird. Dabei erstarrt die Schmelze zu einem festen Strang mit tiefblauer Farbe, welche durch den reversiblen Farbtonwechsel wieder zurückkehrt. Der Strang wird unmittelbar einer Granuliereinrichtung (Häcksler mit Rotationsmesser) zugeführt und in ein Zylindergranulat zu Pellets der Länge 4 mm bei 3 mm Durchmesser granuliert. Es ist ebenso möglich mit anderen Düsengeometrien und Granulierwerken (Bandgranulierung, Kopfgranulierung) zu würfel- oder linsenförmigen Pellets zu gelangen. Ebenso kann die aus dem Extruder austretende Schmelze in Wannen zu Blöcken von z.B. 30 x 30 x 50 cm Kantenlänge erstarren und die Blöcke können dann anschließend unter Kühlung zu Bruchgranulat vermahlen werden, so dass eine Korngrößenverteilung unregelmäßig geformter Pellets von 2 bis 10 mm entsteht. Der in dieser Weise hergestellte Masterbatch enthält gemäß Rezeptierung 50 % Gew-Anteile des Mikrokapsel-Farbstoffes.

Zur Herstellung des thermoplastischen Verbandmaterials wird als thermoplastischer Kunststoff wiederum CAPA 640 in Form weisser Pellets verwendet. Zur Vorbereitung der Einfärbung wird in einem Mischer eine Mischung von 95 Gew-% des weissen Granulats mit 5 Gew-% des obigen Masterbatches hergestellt und homogenisiert. Das Masterbatch-Granulat kann auch über eine übliche gravimetrische Dosiereinheit dem weissen Kunstoff-Granulat kontinuierlich zugemischt werden. Diese Mischung wird über einen Einschneckenextruder an der Beschichtungsanlage bei 140 °C aufgeschmolzen und über eine geeignete Schlitzdüse als farbloser Film ausgetragen, welcher unmittelbar auf die erste Textilbahn aufgelegt wird. Danach wird die zweite textile Bahn aufgelegt und das erhaltene Komposit über Kühlwalzen geführt. Das erhaltene thermoplastische Verbandmaterial wechselt beim Abkühlen den Farbton von farblos wiederum nach blau. Die Bahnware wird zu 2,50 m langen Streifen der Breite 10 cm geschnitten und zu Binden gewickelt

Zur Anwendung werden die Binden im Wasserbad bei 70°C 7 min erwärmt, wobei der Farbton der Kunststoffbeschichtung von blau nach transparent wechselt und die Binde insgesamt weiss (Grundton des Textils) erscheint. Dieser Farbtonwechsel zeigt die vollständige thermische Aktivierung der Binde an, die nunmehr thermoplastisch verformungsfähig ist und damit für den eigentlichen Gebrauch, die Applikation am Körperteil, vorbereitet ist. In diesem Zustand ist das Material darüber hinaus zumindest auf sich selbst haftend. Beim Anwickeln am Körperteil verkleben die einzelnen Lagen miteinander zu einem Lagenverbund. Innerhalb von 10 min kühlt der Verband langsam ab und verfestigt sich wieder, wobei bei Unterschreiten der Umschlagtemperatur des mikroverkapselten thermochromen Farbstoffs der blaue Farbton kontinuierlich wieder erscheint und somit optisch die Verfestigung des Verbandes und die einsetzende Stützwirkung/Stabilisierung für das versorgte Körperteil angezeigt wird. Nach vollständiger Abkühlung auf Raumtemperatur weist der fertige Stützverband den ursprünglichen blauen Farbton der Ausgangsbinde auf. Der Farbtonwechsel blau - weiss - blau erfolgt erneut jeweils reversibel, wenn der kalte Stützverband thermisch nachgearbeitet wird (Fön, Heißluftpistole), um z.B. die Kanten zu glätten bzw. die Form des Verbandes zu ändern und dabei die Umschlagtemperatur des mikroverkapselten thermochromen Farbstoffs zunächst überschritten und beim Abkühlen wieder unterschritten wird. Damit erhält der Anwender ein optisches Signal anhand des jeweiligen Farbtones, welches ihm den Aktivierungszustand des thermoplastischen Verbandmaterials und damit die Formbarkeit/Plastizität beziehungsweise Steifheit anzeigt. In gleicher Weise erhält der Patient anhand des Farbtonwechsels ein optisches Signal, wenn der Verband z.B. bei zu hoher Temperatureinwirkung (Sonnenbad, Sauna) erweicht und Gefahr droht, dass die Stützwirkung verloren geht.

### Beispiel 2: Dosierung über Flüssigfarbe

Zur Herstellung der Flüssigfarbe wird der mikroverkapselte Farbstoff als Pigmentpulver in einer inerten Flüssigkeit, beispielsweise Mineralöl, Esteröl, Fettsäureester, Fettalkohole, Polyethylenglyokole, etc. mit Hilfe eines Dispergators unter Zusatz von Hilfsstoffen wie Tensiden oder Stabilisatoren, Thixotropiermitteln in eine homogene Dispersion überführt, so dass der mikroverkapselte thermochrome Farbstoff in möglichst feinverteilter Form, d.h. frei von Pigmentaggregationen, in der Flüssigkeit verteilt ist. Dabei sind die Rührgeschwindigkeiten und Scherbedingungen so einzustellen, dass die Hülle der Mikrokapseln nicht beschädigt wird und die Kapsel als solche intakt bleibt. Der Gewichtsanteil der Farbstoff-Mikrokapseln in der Flüssigfarbe wird zweckmäßig zwischen 20 - 70 Gew.-% eingestellt. Derartige Flüssigfarben mit mikroverkapselten thermochromen Farbstoffen neigen dennoch zur Sedimentation und müssen jeweils vor Gebrauch aufgerührt werden, um eine ausreichende Homogenität sicherzustellen.

In der eben beschriebenen Weise werden folgende Flüssigfarben-Zubereitungen F1 und F2 hergestellt
- F1:: 30 Gewichtsteile eines mikroverkapselten Blaufarbstoffs (CHROMAZONE - Pigment, der Firma TMC - Thermographic Measurements Ltd., Flintshire (UK), Umschlagtemperatur 47 °C , Hysteresebereich 5,4 K ) werden in 70 Gewichtsteilen n-Octylcaprylat dispergiert, so dass eine 30 % ige Flüssigfarbe des mikroverkapselten thermochromen Farbstoffs resultiert (Farbton: Tiefblau).
- F2:: 50 Gewichtsteile eines mikroverkapselten Rotfarbstoffes (CHROMAZONE - Pigment, der Firma TMC - Thermographic Measurements Ltd., Flintshire (UK), Umschlagtemperatur 31 °C , Hysteresebereich 7,5 K ) werden in 50 Gewichtsteilen n-Octylcaprylat dispergiert, so dass eine 50 %-ige Flüssigfarbe des mikroverkapselten thermochromen Farbstoffs resultiert(Farbton: Tiefrot).

Die beiden Zubereitungen F1 und F2 werden in separate Druckbehälter gefüllt und durch Anwendung von Luft-Überdruck jeweils durch einen separaten Druckschlauch gepumpt, an dessen Ende jeweils ein Nadelventil sitzt, welches mittels Magnetschalter geöffnet und geschlossen werden kann. Durch den anstehenden Überdruck tritt bei geöffnetem Ventil ein gleichmäßiger Strom von Flüssigfarbe aus.

Zur Beschichtung des Textilträgers wird hier eine zu der in Beispiel 1 beschriebenen Einrichtung analoge Einrichtung eingesetzt. Die Einfärbung des weißen thermoplastischen Kunststoffes CAPA 640 erfolgt wie folgt: Die beiden Nadelventile mit Verbindung zu den Vorrats-Druckbehältern der Flüssigfarbe sind nebeneinander direkt in den Extruderkopf eingebaut, welcher zum Aufschmelzen des weißen Kunststoffes benutzt wird. Durch eine elektronische Steuereinheit können die Nadelventile separat angesteuert werden, so dass jeweils über die Taktzeiten von Öffnen und Schließen des Nadelventils ein definierter Flüssigfarben-Strom in den Strom der weißen Kunststoffschmelze abgegeben wird, d.h. ein mengendosierter Eintrag der jeweiligen Flüssigfarbe in den Kunststoffstrom möglich ist.

Die Steuerung wird im vorliegenden Beispiel so eingestellt, dass im Zeitmittel zu 98 g Kunststoff jeweils 1 g Flüssigfarbe F1 und 1 g Flüssigfarbe F2 zudosiert wird. Die Flüssigfarben verteilen sich durch die rotierende Extruderschnecke in homogener Form in der 140°C heißen Kunststoffschmelze. Da die Umschlagtemperatur des mikroverkapselten thermochromen Farbstoffs überschritten ist, ist die am Ende des Extruders aus der Schlitzdüse austretende Schmelze wiederum transparent.

Das analog zu Beispiel 1 daraus hergestellte thermoplastische Verbandmaterial weist im abgekühlten Zustand (bei Raumtemperatur 22 °C) einen violetten Farbton auf, der sich als subtraktive Mischfarbe aus rot und blau ergibt.

Zur Anwendung werden die aus der beschichteten Textilbahn gefertigten Binden im Wasserbad bei 70°C 7 min erwärmt, wobei der Farbton der Kunststoffbeschichtung von violett über kurzzeitig blau nach transparent wechselt und die Binde insgesamt weiß (Grundton des Textils) erscheint. Dieser Farbtonwechsel zeigt die vollständige thermische Aktivierung der Binde an, die nunmehr thermoplastisch verformungsfähig ist und damit für den eigentlichen Gebrauch, die Applikation am Körperteil, vorbereitet ist. Beim Anwickeln am Körperteil verkleben die Lagen miteinander und mit diesem Lagenverbund kühlt der Verband langsam ab und verfestigt sich wieder, wobei bei Unterschreiten der Umschlagtemperatur des mikroverkapselten thermochromen Farbstoff F1 bei 47 °C zunächst der blaue Farbton kontinuierlich wieder erscheint und somit optisch die Verfestigung des Verbandes und die einsetzende Stützwirkung/Stabilisierung für das versorgte Körperteil angezeigt wird. Bei weiterer Abkühlung in Richtung Raumtemperatur kehrt bei 31°C die rote Grundfarbe des mikroverkapselten thermochromen Farbstoffs F2 zurück, was den bisherigen blauen Farbton in den violetten Mischton wechseln lässt. Nach vollständiger Abkühlung weist der fertige Stützverband bei Raumtemperatur 22°C wieder den ursprünglichen violetten Farbton der Ausgangsbinde auf.

Der Farbtonwechsel violett - blau - weiss - blau - violett erfolgt erneut jeweils reversibel bei Aufwärm-/Abkühlzyklen und zeigt das jeweilige Temperaturniveau an, in dem sich der Stützverband momentan befindet (innerhalb des Hysteresebereichs der jeweiligen Umschlagstemperaturen der mikroverkapselten thermochromen Farbstoffe). Durch den mehrfachen Farbwechsel ist eine noch genauere Beurteilung des jeweiligen Zustandes des thermoplastischen Verbandes möglich. Der Farbton blau signalisiert die gerade beginnende Erstarrung der zuvor applizierten Binde und der bei weiterer Abkühlung erfolgende Farbumschlag nach violett zeigt den Zustand an, wenn die maximale Endfestigkeit/Stabilität des Verbandes erreicht ist. Bei der thermischen Nacharbeit mit Fön etc. erhält der Anwender so durch den mehrfachen Farbtonwechsel detaillierte Informationen anhand des jeweiligen Farbtones über das Temperaturniveau und den Festigkeitszustand des thermoplastischen Verbandmaterials und damit eine graduelle Anzeige der Formbarkeit/Plastizität bzw. des Stabilitätsgrades. In gleicher Weise erhält der Patient anhand des mehrfachen Farbtonwechsels eine optische Anzeige, welche ihm als Warn- und Eingriffsgrenze dienen kann, wenn der Verband z.B. bei zu hoher Temperatureinwirkung (Sonnenbad, Sauna) erweicht und Gefahr droht, dass die Stützwirkung verloren geht.

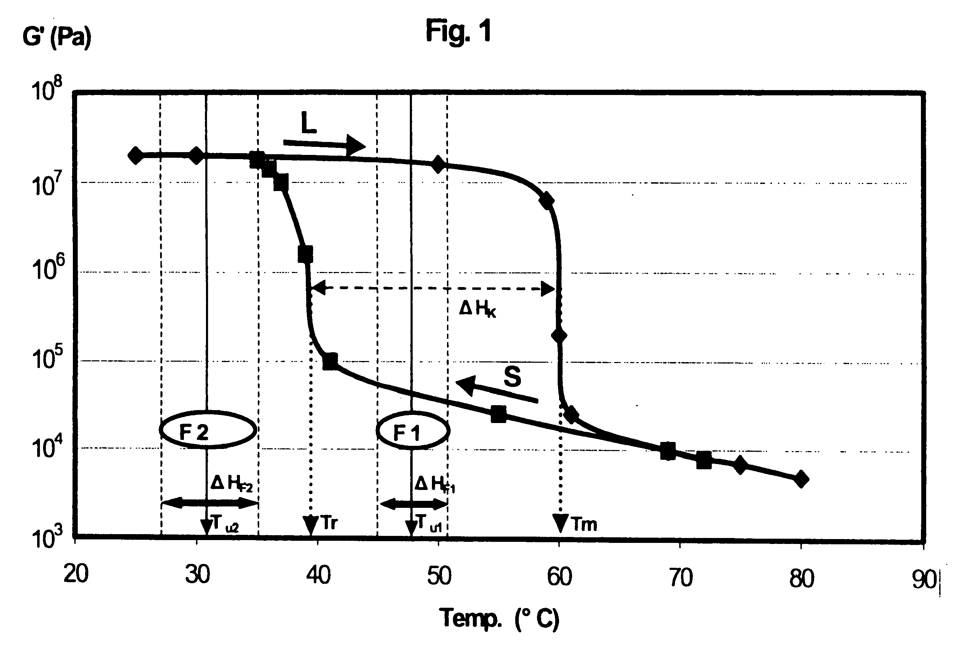

Die grafische Darstellung in Figur 1 erläutert die Zusammenhänge des Beispiels 2 und die Erfindung nochmals näher. Zur Charakterisierung der viskoelastischen Eigenschaften des thermoplastischen Kunststoffes über den gesamten Temperaturbereich der Phasenumwandlung findet die dynamisch-mechanische Analyse Anwendung, wobei sich der Kunststoff zwischen zwei planparallelen Platten befindet, die mit einem mit einem Drehschwingungssystem (Piezo-Rotary-Vibrator) in Schwingung versetzt werden. Durch Messung der Scherspannungen und der zugeordneten Kräfte wird der komplexe Schermodul G* ermittelt, der sich aus dem Speichermodul G' (elastischer Anteil) und dem Verlustmodul G" (viskoser Anteil) zusammensetzt, die über den Verlustwinkel tan δ (Phasenverschiebung) miteinander verknüpft sind. Die Figur 1 zeigt den temperaturabhängigen Verlauf des in dieser Weise ermittelten Speichermoduls G' (elastischer Anteil des Schermoduls) im Temperaturbereich 20 °C bis 80 °C.
Bei Raumtemperatur (25 °C) liegt der Kunststoff in fester Form vor und G' weist Werte der Größenordnung 10⁷ Pascal(Pa) auf. Beim Erwärmen folgt der Schermodul dem Kurvenverlauf L, wobei der Phasenübergang fest - flüssig bei der Schmelztemperatur Tm durch ein deutliches Absinken des Schermoduls auf ca. 10⁴ Pa gekennzeichnet ist, welcher bis zur Temperatur von 80 °C für die Kunststoffschmelze nur noch geringfügig weitersinkt. Beim anschließenden Abkühlen der Schmelze folgt der Schermodul nunmehr dem Kurvenverlauf S, wobei bei der Erstarrungstemperatur Tr wiederum ein deutlicher Anstieg des Schermoduls von 10⁵ auf 10⁷ Pa zu beobachten ist. Die Abweichung der Aufwärmkurve L und Abkühlkurve S kennzeichnet die Hysterese, wobei sich der Hysteresebereich des thermoplastischen Kunststoffes aus der Differenz ΔH_{K} = Tm - Tr ergibt.

In Fig. 1 sind weiterhin die im Beispiel 2 zitierten mikroverkapselten thermochromen Farbstoffe F1 (blauer Farbton) und F2 (roter Farbton) mit den jeweiligen Umschlagtemperaturen Tᵤ₁ und Tᵤ₂ sowie den zugehörigen Hysteresebereichen ΔH_{F1} und ΔH_{F2} in die Figur eingetragen. Die Farbsättigung des thermochromen Farbstoffes ist jeweils an der unteren Grenztemperatur des Hysteresebereiches maximal bzw. an der oberen Grenztemperatur minimal (transparenter Zustand). An Hand der Graphik kann der erfindungsgemäße Effekt nachvollzogen werden, dass beim Erwärmen des mit F1 und F2 eingefärbten thermoplastischen Kunststoffes (Kurvenverlauf L) der violette Grundfarbton ab ca. 35 °C durch den erfolgenden Farbumschlag des Farbstoffs F2 zunächst ab ca. 35 °C in den blauen Farbton (Grundton von F1) wechselt, welcher dann bei weiterer Erwärmung ab ca. 50 °C erst durch den Farbumschlag des Farbstoffes F1 transparent wird. Die Transparenz bleibt während des Schmelzvorgangs auch für den flüssigen Kunststoff bei Temperaturen bis 80 °C erhalten. Beim Abkühlen (Kurvenverlauf S) beginnt der Farbumschlag transparent ► blau im noch geschmolzenen Zustand des thermoplastischen Kunststoffes bei Erreichen des Hysteresebereiches ΔH_{F1} von F1, welcher etwa in der Mitte des Hysteresebereiches ΔH_{K} des thermoplastischen Kunststoffes liegt. Dieser erste Farbumschlag signalisiert die beginnende Erstarrung des thermoplastischen Kunststoffes, der dann auch nach Erstarrung, also im festen Zustand, in diesem Temperaturbereich einen blauen Farbton aufweist. Bei weiterer Abkühlung beginnt bei Erreichen des Hysteresebereiches ΔH_{F2} von F2, welcher unterhalb der Erstarrungstemperatur Tr liegt, der Farbumschlag blau ► violett (ausgelöst durch den Farbwechsel transparent ► rot von F2). Dieser zweite Farbumschlag signalisiert die vollständige Rekristallisation beim Erstarren des thermoplastischen Kunststoffs, und damit auch im fertigen thermoplastischen Verbandstoff die maximale Stützwirkung in der Funktionsphase beim Tragen des Verbandes.

Ein wesentliches Merkmal der Erfindung ist, dass die Farbstoffe F1, F2 hinsichtlich der Lage der Umschlagtemperaturen Tu sowie der Hysteresebereiche beliebig auf der Temperaturskala platziert werden können und damit auf die Hystereseeigenschaften des thermoplastischen Kunststoffes und die beabsichtigte Signalwirkung des Farbwechsels bzw. Korrelation mit den viskoelastischen Eigenschaften eingestellt werden können.

Auf die beschriebene Weise kann besonders einfach eine Gefährdung der Patienten durch toxische Farbstoffe verhindert werden und insbesondere bei Verwendung von thermochromen Farbstoffen die Zustandsänderung, aber auch weitere Verarbeitungs- und Materialparameter einem Anwender beziehungsweise Patienten signalisiert werden.

## Patentansprüche

1. Verbandmaterial, umfassend einen thermoplastischen Kunststoff, wobei der thermoplastische Kunststoff auf einer ersten Textilbahn aufgebracht ist, **dadurch gekennzeichnet, dass** der thermoplastische Kunststoff einen ersten mikroverkapselten Farbstoff enthält.

2. Verbandmaterial nach Anspruch 1**, dadurch gekennzeichnet, dass** der erste mikroverkapselte Farbstoff ein thermochromer Farbstoff ist.

3. Verbandmaterial nach Anspruch 1 oder 2, **dadurch gekennzeichnet, dass** der thermoplastische Kunststoff mindestens einen zweiten Farbstoff beinhaltet.

4. Verbandmaterial nach Anspruch 3, **dadurch gekennzeichnet, dass** der mindestens zweite Farbstoff ein mikroverkapselter, und insbesondere thermochromer Farbstoff ist.

5. Verbandmaterial nach einem der vorangehenden Ansprüche, **dadurch gekennzeichnet, dass** der mindestens erste und/oder die weiteren thermochromen Farbstoffe hinsichtlich ihrer Umschlagpunkte Tu mit thermischen und/oder mechanischen Material- und/oder Verarbeitungsparametern des thermoplastischen Kunststoffs korrelieren.

6. Verbandmaterial nach einem der vorangehenden Ansprüche, **dadurch gekennzeichnet, dass** weitere Textilbahnen und/oder thermoplastische Kunststoffschichten vorgesehen und zu einem Verbund angeordnet sind.

7. Verfahren zum Herstellen eines Verbandmaterials nach einem der vorangehenden Ansprüche mit den folgenden Schritten:
- Bereitstellen einer Textilbahn,
- Einmischen eines mindestens ersten mikroverkapselten Farbstoffes und/oder einer Farbzubereitung umfassend einen ersten mikroverkapselten Farbstoff in einen thermoplastischen Kunststoff zur Herstellung einer Beschichtungsmasse,
- Beschichten der Textilbahn mit der Beschichtungsmasse,
- vorzugsweise Auflegen einer weiteren Textilbahn auf die Beschichtungsmasse,
- Konfektionieren des Verbandsmaterials.

8. Verfahren nach Anspruch 7, **dadurch gekennzeichnet, dass** die Farbzubereitung durch Zugabe des mikroverkapselten Farbstoffs zu einem Trägerkunststoff hergestellt wird, die Mischung geschmolzen, homogenisiert und anschließend nach Abkühlen granuliert wird.

9. Verfahren nach Anspruch 8, wobei der Schmelzpunkt des Trägerkunststoffes in einem Bereich von 10 K unterhalb bis 60 K oberhalb des Schmelzpunktes des thermoplastischen Kunststoffes liegt.

10. Verfahren nach Anspruch 7 **dadurch gekennzeichnet, dass** die Farbzubereitung durch Zugabe des mikroverkapselten Farbstoffs zu einer inerten Flüssigkeit und Homogenisieren der Mischung hergestellt wird.

11. Verfahren nach einem der Ansprüche 7 bis 10, **dadurch gekennzeichnet, dass** zur Herstellung der Beschichtungsmasse mindestens ein zweiter Farbstoff und/oder mindestens eine zweite Farbzubereitung und/oder eine Farbzubereitung umfassend mehrere Farbstoffe, eingemischt werden.

12. Verwendung eines Verbandmaterials nach einem der Ansprüche 1 bis 6 zur Herstellung eines orthopädischen Stützverbandes.

13. Binde, insbesondere zur Herstellung eines orthopädischen Stützverbandes umfassend ein Verbandmaterial nach einem der Ansprüche 1 bis 6.

14. Orthopädischer Stützverband umfassend ein Verbandmaterial nach einem der Ansprüche 1 bis 6.

## Claims

1. A dressing material, comprising a thermoplastic polymer, the thermoplastic polymer being applied to a first textile web, **characterized in that** the thermoplastic polymer comprises a first micro-encapsulated dye.

2. The dressing material according to claim 1, **characterized in that** the first micro-encapsulated dye is a thermochromic dye.

3. The dressing material according to claim 1 or 2, **characterized in that** the thermoplastic polymer comprises at least a second dye.

4. The dressing material according to claim 3, **characterized in that** at least the second dye is a micro-encapsulated and particularly a thermoplastic dye.

5. The dressing material according to any one of the preceding claims, **characterized in that** at least the first and/or the further thermochromic dyes with respect to their turning points Tu correlate with thermal and/or mechanical material and/or processing parameters of the thermoplastic polymer.

6. The dressing material according to any one of the preceding claims, **characterized in that** further textile webs and/or thermoplastic polymer layers are provided and may form a composite.

7. A method for producing a dressing material according to any one of the preceding claims, comprising the following steps:
- providing a textile web,
- mixing of at least a first micro-encapsulated dye and/or a colour preparation comprising a first micro-encapsulated dye in a thermoplastic polymer to produce a coating compound,
- coating the textile web with the coating compound,
- preferably applying a further textile web onto the coating compound,
- customizing the dressing material.

8. The method according to claim 7, **characterized in that** the colour preparation is produced by adding the micro-encapsulated dye to a carrier polymer, melting mixture is, homogenizing and then granulating it after cooling.

9. The method according to claim 8, wherein the melting of the carrier polymer is in a range of 10 K below to 60 K above the melting point of the thermoplastic polymer.

10. The method according to claim 7, **characterized in that** the colour preparation is produced by adding the micro-encapsulated dye to an inert fluid and by homogenizing the mixture.

11. The method according to any one of the claims 7 to 10, **characterized in that** for producing the coating compound at least a second dye and/or at least a second colour preparation and/or a colour preparation comprising a plurality of dyes are mixed in.

12. Use of the dressing material according to any one of the claims 1 to 6 for producing an orthopaedic support dressing.

13. A bandage, particularly for producing an orthopaedic support dressing, comprising a dressing to any one of the claims 1 to 6.

14. An orthopaedic support dressing, comprising a dressing material according to any one of the claims 1 to 6.

## Revendications

1. Matériau de bandage, comprenant un plastique thermoplastique, le plastique thermoplastique étant placé sur une première bande textile, **caractérisé en ce que** le plastique thermoplastique comprend un premier colorant micro-encapsulé.

2. Matériau de bandage selon la revendication 1, **caractérisé en ce que** le premier colorant micro-encapsulé est un colorant thermochrome.

3. Matériau de bandage selon les revendications 1 ou 2, **caractérisé en ce que** le plastique thermoplastique contient au moins un deuxième colorant.

4. Matériau de bandage selon la revendication 3, **caractérisé en ce que** le, au moins, deuxième colorant est un colorant micro-encapsulé et en particulier thermochrome.

5. Matériau de bandage selon l'une des revendications précédentes, **caractérisé en ce que** le, au moins, premier et/ou les autres colorants thermochromes sont en corrélation avec les paramètres thermiques et/ou mécaniques de matériau et/ou de fabrication du plastique thermoplastique en ce qui concerne leur point final de réaction Tu.

6. Matériau de bandage selon l'une des revendications précédentes, **caractérisé en ce que** d'autres bandes textiles et/ou couches plastiques thermoplastiques sont prévues et agencées de manière à former un composite.

7. Procédé de fabrication d'un matériau de bandage selon l'une des revendications précédentes comportant les phases suivantes :
- approvisionnement d'une bande textile,
- incorporation d'un, au moins, premier colorant micro-encapsulé et/ou d'une préparation colorante comprenant un premier colorant micro-encapsulé dans un plastique thermoplastique pour la fabrication d'une substance de revêtement,
- enduction de la bande textile avec la substance de revêtement,
- de préférence mise en place d'une autre bande textile sur la substance de revêtement,
- confection du matériau de bandage.

8. Procédé selon la revendication 7, **caractérisé en ce que** la préparation colorante est élaborée par adjonction du colorant micro-encapsulé à une matière plastique porteuse ; le mélange est fondu, homogénéisé et pour finir réduit en granulés après refroidissement.

9. Procédé selon la revendication 8, le point de fusion de la matière plastique porteuse étant situé entre 10 K en dessous et 60 K au-dessus du point de fusion du plastique thermoplastique.

10. Procédé selon la revendication 7, **caractérisé en ce que** la préparation colorante est réalisée par adjonction du colorant micro-encapsulé à un liquide inerte et homogénéisation du mélange.

11. Procédé selon l'une des revendications 7 à 10, **caractérisé en ce que**, pour l'élaboration de la substance de revêtement, il est incorporé au moins un deuxième colorant et/ou au moins une deuxième préparation colorante et/ou une préparation colorante contenant plusieurs colorants.

12. Utilisation d'un matériau de bandage selon l'une des revendications 1 à 6 pour la réalisation d'un bandage de soutien orthopédique.

13. Bandage, notamment pour la réalisation d'un bandage de soutien orthopédique, comprenant un matériau de bandage selon l'une des revendications 1 à 6.

14. Bandage de soutien orthopédique comprenant un matériau de bandage selon l'une des revendications 1 à 6.
